**Europäisches Patentamt**

**European Patent Office**

**Office européen des brevets**

(11) Veröffentlichungsnummer: **0 437 788 B1**

## (12) EUROPÄISCHE PATENTSCHRIFT

(45) Veröffentlichungstag der Patentschrift: **01.02.95**

(51) Int. Cl.⁶: **B22F 9/08**

(21) Anmeldenummer: **90124870.8**

(22) Anmeldetag: **20.12.90**

(54) **Verfahren zur Herstellung pulverförmiger Aluminiumlegierungen.**

(30) Priorität: **19.01.90 DE 4001484**

(43) Veröffentlichungstag der Anmeldung:
**24.07.91 Patentblatt 91/30**

(45) Bekanntmachung des Hinweises auf die
Patenterteilung:
**01.02.95 Patentblatt 95/05**

(84) Benannte Vertragsstaaten:
**BE CH DE FR GB IT LI NL SE**

(56) Entgegenhaltungen:
**DE-A- 3 402 983**
**US-A- 3 839 011**
**US-A- 4 348 270**

(73) Patentinhaber: **BAYER AG**

**D-51368 Leverkusen (DE)**

Patentinhaber: **H.C. Starck GmbH & Co. KG**
**Im Schleeke 78-91**
**D-38642 Goslar (DE)**

(72) Erfinder: **Birkenstock, Udo, Dr.**
**Schneppersdelle 8**
**W-4030 Ratingen 8 (DE)**
Erfinder: **Scharschmidt, Jürgen, Dr.**
**Holzapfelweg 1**
**W-4150 Krefeld-Traar (DE)**
Erfinder: **Kunert, Peter, Dipl.-Ing.**
**Hammermattstrasse 15**
**W-7887 Laufenburg (DE)**
Erfinder: **Meinhardt, Helmut, Dr.**
**Lauberstrasse 21**
**W-7886 Murg-Hänner (DE)**
Erfinder: **Häusel, Paul**
**Dürrenbachstrasse 14**
**W-7888 Rheinfelden-Baden (DE)**
Erfinder: **Meier Paul**
**Am Römerfeld 14**
**W-4156 Willich 4 (DE)**

## Beschreibung

Die vorliegende Erfindung betrifft ein besonders vorteilhaftes Verfahren zur Herstellung von pulverförmigen Aluminiumlegierungen, die eine katalytische Wirkung aufweisen oder Vorläufer von Katalysatoren sind.

Besondere Bedeutung als Katalysatoren für Hydrierungen haben sog. Raney-Metalle erlangt. Man kann diese beispielsweise herstellen, indem man zunächst eine Legierung zusammenschmilzt, die Aluminium und ein Nebengruppenelement, z.B. Nickel, enthält, diese Legierung, z.B. in Kugelmühlen, fein zerkleinert und durch Herauslösen des Aluminiums mit Alkalien das ursprünglich eingesetzte Nebengruppenelement in katalytisch aktiver Form erhält (siehe z.B. Chemiker Zeitung 1975, S. 446 bis 452).

US-A-3 839 011 zeigt ein Verfahren zur Herstellung von Aluminiumlegierungen, die eine katalytisch Wirkung aufweisen, wobei die flüssige Legierung in einem Wasserstrom zertrümmert wird. Die so hergestellten Partikel bestehen im wesentlichen aus dünnen, zusammengefalteten Blättchen, die noch weiter zerkleinert werden.

Wichtige katalytisch wirksame Aluminiumlegierungen sind z.B. Aluminium-Zink-Legierungen, die man z.B. bei der Alkylierung von m-Phenylendiaminen verwenden kann (siehe DE-OS 3 402 983).

Die katalytische Wirkung von Aluminiumlegierungen und die katalytische Wirkung von aus Aluminiumlegierungen hergestellten Katalysatoren hängt u.a. von der qualitativen und quantitativen Zusammensetzung der jeweiligen Aluminiumlegierung, aber auch von deren Korngröße und Teilchenstruktur ab.

Bisher kann man nicht alle denkbaren Kombinationen von qualitativer und quantitativer Zusammensetzung mit verschiedenen Korngrößen und verschiedenen inneren und äußeren Teilchenstrukturen realisieren. Beispielsweise lassen sich Aluminiumlegierungen mit Aluminiumgehalten von über 50 Gew.-% nicht mehr problemlos vermahlen. Durch neuartige Zerkleinerungsmethoden könnten die oben angegebenen Legierungssysteme mit Teilchenstrukturen realisiert werden, die bisher nicht erhalten werden konnten.

Es wurde nun ein Verfahren zur Herstellung von pulverförmigen Aluminiumlegierungen gefunden, die eine katalytische Wirkung aufweisen oder als Vorläufer von Katalysatoren dienen, das dadurch gekennzeichnet ist, daß man eine flüssige Aluminiumlegierung bei einer Temperatur von 50 bis 500 °C über ihrem Schmelzpunkt mit Wasser und/oder einem Gas verdüst und abkühlt und dann gegebenenfalls noch vorhandenes Wasser ganz oder bis auf einen Gehalt von unter 1 Gew.-% entfernt.

In das erfindungsgemäße Verfahren können Aluminiumlegierungen mit beliebigem Gehalt an Aluminium eingesetzt werden. Vorzugsweise beträgt dieser zwischen 50 und 99 Gew.-%, z.B. 55 bis 95 oder 58 bis 90 Gew.-%. Besonders bevorzugt sind Aluminiumlegierungen mit 60 bis 90 Gew.-% Aluminiumgehalt. Neben Aluminium kann die Legierung ein oder mehrere weitere Metalle enthalten. Vorzugsweise handelt es sich dabei um Nebengruppenelemente wie Scandium, Titan, Vanadin, Chrom, Mangan, Eisen, Kobalt, Nickel, Kupfer, Zink, Yttrium, Zirkon, Niob, Molybdän, Ruthenium, Rhodium, Palladium, Silber, Cadmium, Lanthan, Cer, Hafnium, Tantal, Wolfram, Rhenium, Osmium, Iridium, Platin, Gold und die Lanthaniden. Bevorzugt sind Legierungen, die neben Aluminium Nickel, Kobalt, Molybdän, Kupfer, Titan, Eisen und/oder Tantal oder neben Aluminium Zink enthalten. Die Aluminiumlegierungen können auch sogenannte Promotoren enthalten. Beispielsweise kann eine im wesentlichen aus Aluminium und Nickel bestehende Legierung als Promotoren weitere Nebengruppenelemente, beispielsweise Eisen, Kobalt, Mangan, Molybdän und/oder Kupfer enthalten. Der Gehalt an Promotormetallen kann beispielsweise 0,01 bis 50 Gew.-%, bezogen auf die Gesamtlegierung, betragen. Vorzugsweise beträgt dieser Gehalt 0,05 bis 10 Gew.-%.

In das erfindungsgemäße Verfahren einsetzbare Aluminiumlegierungen können, wie beschrieben, qualitativ und quantitativ auf sehr vielfältige Weise zusammengesetzt sein. Es muß lediglich sichergestellt sein, daß die einzelnen Komponenten auch tatsächlich eine Legierung bilden, d.h. daß alle Komponenten im schmelzflüssigem Zustand miteinander mischbar sind.

Zum Verdüsen wird die Aluminiumlegierung auf eine Temperatur von 50 bis 500 °C, vorzugsweise 150 bis 250 °C, über ihrem Schmelzpunkt erhitzt. Die Verdüsung kann dann in Vorrichtungen erfolgen, wie sie beispielsweise für die Erzeugung von Metallpulvern für pulvermetallurgische Zwecke üblich sind, beispielsweise in einer Schlitzdüse, in welche die flüssige Aluminiumlegierung zentral und das Verdüsungsmedium konzentrisch dazu eingeführt wird.

Die Verdüsung wird mit Wasser und/oder einem Gas durchgeführt. Wenn man der Verdüsungsvorrichtung die flüssige Aluminiumlegierung in einer Menge von 10 bis 50 kg/min zuführt, so kann man beispielsweise pro kg Aluminiumlegierung 5 bis 15 l Wasser mit einem Druck von beispielsweise 10 bis 500 bar oder beispielsweise pro kg Aluminiumlegierung 0,5 bis 10 $m^3$ Gas mit einem Druck von beispielsweise 2 bis 100 bar einsetzen. Bevorzugt sind folgende Mengenkombinationen: 15 bis 40 kg/min flüssige Aluminiumlegierung, pro kg Aluminiumlegierung 6 bis 12 l Wasser mit einem Druck von 20 bis 400 bar oder 1 bis 8 $m^3$ Gas mit einem Druck von 5 bis 80 bar.

2

EP 0 437 788 B1

Hier und im folgenden bedeutet x m³ Gas, gemessen bei dem in der Verdüsungsapparatur herrschenden Druck.

Als Gase für die Verdüsung kommen beispielsweise solche in Frage, die 1 bis 35 Gew.-% Sauerstoff enthalten. Außer Sauerstoff können z.B. weitgehend oder vollständig inerte Gase vorhanden sein, wie Stickstoff und/oder Edelgase. Vorzugsweise werden in die Verdüsung Gase eingesetzt, die 5 bis 25 Gew.-% Sauerstoff enthalten, besonders bevorzugt Luft. Es können auch Sauerstoff-freie Gase verwendet werden, z.B. Stickstoff oder Argon.

Die Verdüsung mit Wasser ergibt überwiegend spratzige Teilchen mit feiner innerer Gefügestruktur, die Verdüsung mit Gas überwiegend kugelige bis irreguläre Teilchen mit grober Gefügestruktur. Welche Art der Verdüsung und welche dabei anzuwendenden Mengen an Aluminiumlegierung, Verdüsungsmedien und Zusammensetzungen von Verdüsungsmedien für eine bestimmte Aluminiumlegierung und deren Verwendung für einen bestimmten katalytischen Prozeß oder als Vorläufer für einen Katalysator für einen bestimmten katalytischen Prozeß jeweils ein optimales Ergebnis erbringen, kann gegebenenfalls für einen konkreten Einzelfall durch routinemäßige Reihenversuche ermittelt werden.

An die Verdüsung schließt sich eine Abkühlung an. Diese kann man beispielsweise mit Wasser oder einem Gas durchführen. Bei der Verwendung von Wasser kann man die verdüste Aluminiumlegierung im einfachsten Fall in ein ausreichend hohes, mit Wasser gefülltes oder durchströmtes Gefäß von oben her einbringen. Die verdüste Legierung kühlt sich dann während des Absinkens ab. Bei der Verwendung eines Gases, z.B. Luft kann man dieses z.B. im Gegenstrom zu herabfallenden Teilchen der verdüsten Aluminiumlegierung führen.

Die Temperatur der verdüsten Aluminiumlegierung kann nach dem Abkühlen mit Wasser beispielsweise unter 50°C liegen. Vorzugsweise liegt sie dann bei 10 bis 30°C. Nach dem Abkühlen mit Luft kann die Temperatur der verdüsten Aluminiumlegierung beispielsweise unter 100°C liegen. Vorzugsweise liegt sie dann unter 50°C, insbesondere bei 30 bis 48°C.

Aluminiumlegierungen der oben beschriebenen Art, die auf erfindungsgemäße Weise verdüst und abgekühlt werden sind im allgemeinen lagerstabil, wenn ihr Wassergehalt unter 1 Gew.-%, vorzugsweise unter 0,1 Gew.-% liegt. Solche niedrigen Wassergehalte stellen sich im allgemeinen von selbst ein, wenn man die Abkühlung mit trockener Luft vornimmt. Wird die Abkühlung jedoch mit Wasser vorgenommen, so muß die verdüste, abgekühlte Aluminiumlegierung noch getrocknet werden, um ein lagerstabiles Produkt zu erhalten. Man kann dabei so verfahren, daß man ein trockenes Gas in einer Menge von beispielsweise 1 bis 10 m³/kg Aluminiumlegierung einsetzt und gegebenenfalls das zu trocknende Material während der Trocknung umwälzt. Wenn man kein lagerstabiles Produkt benötigt, z.B. weil man es sofort als Katalysator verwenden oder sofort einen Katalysator daraus herstellen will, so kann die Trocknung natürlich unterbleiben.

Die erfindungsgemäß hergestellten pulverförmigen Aluminiumlegierungen zeichnen sich dadurch aus, daß sie besonders aktive Katalysatoren sind oder sich besonders aktive Katalysatoren, z.B. solche vom Raney-Typ, daraus herstellen lassen. Insbesondere lassen sich erfindungsgemäß pulverförmige Aluminiumlegierungen mit hohem Aluminiumgehalt auf einfache Weise herstellen. So können Teilchenstrukturen und Katalysatoren erhalten werden, die bisher nicht zugänglich waren.

Soweit es sich bei den erfindungsgemäß hergestellten pulverförmigen Aluminiumlegierungen um Katalysatoren handelt, beispielsweise um Aluminium-Zink-Legierungen, können diese zur Alkylierung aromatischer Verbindungen, z.B. zur Alkylierung von Phenolen, Anilinen oder Phenylendiaminen verwendet werden. Soweit es sich bei den erfindungsgemäß hergestellten pulverförmigen Aluminiumverbindungen um Vorläufer von Katalysatoren handelt, beispielsweise um Aluminiumlegierungen, die Nickel, Kobalt, Eisen und/oder Mangan enthalten, können daraus, z.B. durch Behandlung mit Alkalien, Katalysatoren hergestellt werden, die sich für Hydrierungen eignen. Beispiele für solche Hydrierungen sind die Umwandlung von ungesättigten Kohlenwasserstoffen in weniger ungesättigte oder gesättigte Kohlenwasserstoffe, die Umwandlung von Nitrilen in Amine und die Überführung von Nitrogruppen, insbesondere an aromatische Systeme gebundene Nitrogruppen, in Aminogruppen.

Beispiele

Beispiel 1

a) Herstellung und Verdüsung einer Legierung

In einem Induktionsofen wurden 60 kg Nickel und 140 kg Aluminium geschmolzen. Nach dem Erreichen der Liquidus-Temperatur wurde die erhaltene Schmelze weiter bis auf 1200°C erhitzt und dann aus dem

3

Induktionsofen in einen beheizten Auslauftrichter mit einem Bodenauslauf von 18 mm Durchmesser gegossen. Der aus diesem Trichter austretende flüssige Metallstrahl wurde mit einem konzentrisch angeordneten ringförmigen Wasserstrahl von 30 bar erfaßt und zerstäubt. Die Verdüsungszeit betrug 10 Minuten, die zugeführte Wassermenge 2 m³. Das verdüste Pulver wurde in einem unterhalb des Verdüsungssystems befindlichen Wassertank von 2 m Höhe aufgefangen und abgekühlt. Die Temperatur der aus dem Tank entnommenen verdüsten Legierung betrug 23°C. Die Restfeuchte (19 Gew.-% Wasser) wurde in einem trockenen, erwärmten Luftstrom von 4000 m³/h innerhalb von 20 Minuten weitestgehend entfernt.

So wurden 195 kg Legierungspulver mit folgender Analyse erhalten: 29,5 Gew.-% Nickel, 69,7 Gew.-% Aluminium, 0,4 Gew.-% Sauerstoff und 0,05 Gew.-% Wasser.

Das erhaltene Pulver zeigte eine überwiegend spratzige Kornform, die erhaltene Kornverteilung war:

```
über  400 µm              5,5 Gew.-%

200 bis 400 µm           28,1 Gew.-%

100 bis 200 µm           41,6 Gew.-%

50 bis 100 µm            18,9 Gew.-%

unter 50 µm               5,9 Gew.-%
```

Das erhaltene Pulver war lagerstabil und konnte problemlos gehandhabt werden.

b) Herstellung eines Katalysators

782 g Natriumhydroxid wurden in einem Becherglas in 3129 g Wasser gelöst. Die Temperatur der so erhaltenen Natriumhydroxidlösung wurde auf 80°C eingestellt. Die Luft über der Lösung wurde gegen Stickstoff ausgetauscht und während der gesamten Reaktionszeit unter einem Stickstoffschleier gearbeitet.

Anschließend wurden 200 g der gemäß a) erhaltenen pulverförmigen Legierung zu der Natriumhydroxid-Lösung in Portionen von jeweils 6 g zugefügt. Die Zugabe der pulverförmigen Legierung erfolgte über einen Zeitraum von 20 Minuten, wobei die Temperatur der Natriumhydroxid-Lösung bei 80 ± 2°C gehalten wurde, um die Schaumbildung nicht zu stark werden zu lassen.

Nachdem die 200 g der pulverförmigen Legierung eingetragen waren, wurde das Reaktionsgemisch noch 30 Minuten bei 80°C gerührt. Anschließend wurde die Mutterlauge vom gebildeten Katalysator abdekantiert und der Katalysator mit einer Lösung von 78 g Natriumhydroxid in 313 g Wasser 5 Minuten lang unter Rühren nachbehandelt. Dann wurde diese Waschlauge durch Dekantieren abgetrennt und der Katalysator mit Wasser bis zu einem pH-Wert von 8,0 gewaschen.

Es wurden 59 g Katalysator (100 %ig), anfallend als wäßriger Schlamm, erhalten.

c) Anwendung des Katalysators

In einem 0,7 l Autoklaven mit Rührer und elektrischer Außenheizung wurden 200 g Phenol und 10 g des gemäß b) erhaltenen Katalysators (100 %ig) bei 230°C und 150 bar Wasserstoffdruck hydriert. In einer Reaktionszeit von 12 Minuten wurde ein Gemisch erhalten, das ohne Berücksichtigung des Reaktionswassers 97,4 Gew.-% Cyclohexanol, 2,3 Gew.-% Cyclohexan und 0,3 Gew.-% sonstige Nebenprodukte enthielt.

Beispiel 2

a) Herstellung und Verdüsung einer Legierung

In einem Induktionsofen wurden 71 kg Nickel, 12,4 kg Eisen und 122,8 kg Aluminium geschmolzen. Nach Erreichen der Liquidustemperatur wurde die erhaltene Schmelze weiter bis auf 1300°C erhitzt und dann aus dem Induktionsofen in einen beheizten Keramikgießtrichter mit einem mittig angeordneten Bodenauslauf gegossen. Der aus dem Bodenauslauf austretende flüssige Metallstrahl von 15 mm Durchmesser wurde mit Druckluft von 6,2 bar, die durch eine konzentrisch angeordnete Ringspaltdüse zugeführt wurde, zerstäubt. Die Verdüsungszeit betrug 11 Minuten, der Luftverbrauch 1000 m³ (gemessen bei 6,2 bar). Das verdüste Pulver wurde unterhalb des Verdüsungssystems in einem Wassertank aufgefangen. Die Temperatur der aus dem Tank entnommenen verdüsten Legierung betrug 21°C.

Das über einer Nutsche vorentwässerte Pulver mit einer Restfeuchte von 18 Gew.-% Wasser wurde in einem Strom trockener und angewärmter Luft von 4200 m³/h innerhalb von 24 Minuten getrocknet. Die Restfeuchte betrug dann nur noch 0,03 Gew.-% Wasser.

So wurden 199,4 kg Legierungspulver mit folgender Analyse erhalten: 33,9 Gew.-% Nickel, 5,72 Gew.-% Eisen, 59,8 Gew.-% Aluminium und 0,39 Gew.-% Sauerstoff.

Das erhaltene Pulver zeigte überwiegend sphärische Kornform und wies folgende Kornverteilung auf:

```
über 400 µm          12,6 Gew.-%

200 bis 400 µm       37,3 Gew.-%

100 bis 200 µm       35,2 Gew.-%

50 bis 100 µm        11,6 Gew.-%

unter 50 µm           3,3 Gew.-%.
```

b) Herstellung eines Katalysators

Das gemäß a) erhaltene Legierungspulver wurde wie in Beispiel 1b) beschrieben in einen Katalysator überführt. Es wurden 79 g Katalysator 100 %ig, anfallend als wäßriger Schlamm, erhalten.

c) Anwendung des Katalysators

In einen 0,7 l-Autoklaven mit Rührer und elektrischer Außenheizung wurden 80 g eines Gemisches aus 65 Gew.-% 2,4- und 35 Gew.-% 2,6-Dinitrotoluol und 240 g Methanol eingebracht und mit 10 g gemäß b) erhaltenem Katalysator (100 %ig) bei 100 °C und 100 bar Wasserstoffdruck hydriert. In einer Reaktionszeit von 14 Minuten wurden die Dinitrotoluole nahezu quantitativ umgesetzt und die entsprechenden Diamine, sowie als Nebenprodukt 0,13 Gew.-% N-Alkylverbindungen erhalten.

Die größere Aktivität dieses Katalysators im Vergleich zu denen des Standes der Technik zeigt sich vor allem in der kürzeren Zeit, die zu seiner Herstellung notwendig ist.

Beispiel 3

a) Herstellung und Verdüsung einer Legierung

In einem Induktionsofen wurden 71 kg Nickel, 12,4 kg Eisen und 122,8 kg Aluminium geschmolzen. Nach Erreichen der Liquidustemperatur wurde die erhaltene Schmelze weiter bis auf 1300 °C erhitzt und dann über einen Keramikgießtrichter mit einem mittig angeordneten Bodenauslauf einem Verdüsungssystem zugeführt. Der aus dem Bodenauslaufstein des Trichters austretende flüssige Metallstrahl von 15 mm Durchmesser wurde mit Druckluft von 6,2 bar, die durch eine konzentrisch angeordnete Ringspaltdüse zugeführt wurde, verstäubt, wobei unterhalb der Ringspaltdüse aus einem Sprühring Wasser in das verdüste Pulver eingesprüht wurde. Die Verdüsungszeit betrug 11 Minuten, der Luftverbrauch 1000 m³ (gemessen bei 6,2 bar). Das verdüste Pulver wurde unterhalb des Verdüsungssystems in einem Wassertank aufgefangen. Die Temperatur des dem Tank entnommenen Pulvers betrug 21 °C.

Das über eine Nutsche vorentwässerte Pulver mit einer Restfeuchte von 18 Gew.-% Wasser wurde in einem Luftstrom von 4200 m³/h aus vorgewärmter trockener Luft innerhalb von 24 Minuten getrocknet. Die Restfeuchte des getrockneten Materials betrug nur noch 0,03 Gew.-% Wasser.

So wurden 199,4 kg Legierungspulver mit folgender Analyse erhalten: 33,9 Gew.-% Nickel, 5,72 Gew.-% Eisen, 59,8 Gew.-% Aluminium und 0,39 Gew.-% Sauerstoff.

Das erhaltene Pulver zeigte überwiegend sphärische Kornform und wies folgende Kornverteilung auf:

| | |
|---|---|
| über 400 µm | 12,6 Gew.-% |
| 200 bis 400 µm | 37,3 Gew.-% |
| 100 bis 200 µm | 35,2 Gew.-% |
| 50 bis 100 µm | 11,6 Gew.-% |
| unter 50 µm | 3,3 Gew.-%. |

b) Herstellung eines Katalysators

Das gemäß a) erhaltene Legierungspulver wurde wie in Beispiel 1b) beschrieben in einen Katalysator überführt. Es wurden 79 g Katalysator 100 %ig, anfallend als wäßriger Schlamm, erhalten.

c) Anwendung des Katalysators

Mit dem gemäß b) erhaltenen Katalysator wurde in gleicher Weise wie in Beispiel 2c) ein Dinitrotoluolgemisch hydriert. Die Ergebnisse entsprachen denen des Beispiel 2c).

Beispiel 4

a) Herstellung und Verdüsung einer Legierung

In einem Induktionsofen wurden 2,31 kg Nickel, 2,31 kg Tantal und 18,5 kg Aluminium geschmolzen. Das Gemisch wurde unter 1000 mbar Argon bis zum Liquiduspunkt und anschließend bis auf 1200°C erhitzt und dann in einen vorbeheizten Gießtrichter gegossen, der symmetrisch am Boden eine Auslaßöffnung von 7 mm Durchmesser hatte. Der austretende flüssige Metallstrahl wurde über eine konzentrisch angeordnete Ringspaltdüse mit Argon zerstäubt. Der Gasdruck betrug 6,8 bar, die Verdüsungszeit 90 Sekunden, verbraucht wurden 33,9 m³ Argon (gemessen bei 6,8 bar).
Die Abkühlung der verdüsten Legierung erfolgte in einem Argon-Strom über eine freie Fallhöhe von 4,5 m. Die Temperatur des erhaltenen Pulvers betrug 35°C.
So wurden 19,4 kg Legierungspulver mit folgender Analyse erhalten: 10,1 Gew.-% Nickel, 8,3 Gew.-% Tantal, 0,03 Gew.-% Sauerstoff und 81,5 Gew.-% Aluminium. In diesem Legierungspulver konnte analytisch keine Restfeuchte nachgewiesen werden.
Das erhaltene Pulver zeigte fast ausschließlich sphärische Kornform, die Kornverteilung war wie folgt:

| | |
|---|---|
| über 150 µm | 21,4 Gew.-% |
| 106 bis 150 µm | 13,8 Gew.-% |
| 52 bis 106 µm | 23,2 Gew.-% |
| unter 53 µm | 41,6 Gew.-%. |

b) Herstellung eines Katalysators

Das gemaß a) erhaltene Legierungspulver wurde wie in Beispiel 1b) beschrieben in einen Katalysator überführt. Es wurden 40 g Katalysator 100 %ig, anfallend als wäßriger Schlamm, erhalten.

6

c) Anwendung des Katalysators

Zum Einsatz gelangte ein Autoklav mit 80 ml Flüssigvolumen, versehen mit einem Begasungsrührer, einer Wasserstoffzuführungsleitung, einem Einleitungsrohr für die aromatische Dinitroverbindung, dessen unteres Ende direkt am Begasungsrührer endete, und einem Auslaßventil für überschüssigen Wasserstoff. Das Reaktionsgemisch aus einem aromatischen Diamin und Wasser verlies den Autoklaven durch eine Fritte, die den Katalysator zurückhielt. Diese Apparatur ermöglichte die kontinuierliche Hydrierung von aromatischen Dinitroverbindungen bis zur völligen Erschöpfung des Katalysators. Die Temperatur im Autoklaven wurde durch einen äußeren Heiz- und Kühlkreislauf und eine Kühlschlange im Inneren des Reaktors geregelt.

80 ml eines Gemisches aus (i) einem Gemisch aus 80 Gew.-% 2,4- und 20 Gew.-% 2,6-Diaminotoluol mit (ii) Wasser im Gewichtsverhältnis (i) : (ii) = 63:37, in welchem 1,6 g gemäß b) erhaltenen Katalysator suspendiert vorlagen, wurden in dem Autoklaven vorgelegt. Anschließend wurde der Autoklaveninhalt unter einem Wasserstoffdruck von 15 bar auf 190 bis 200°C aufgeheizt. Bei dieser Temperatur wurden stündlich 64 l Wasserstoff und mittels einer Dosierpumpe 53 g eines Gemisches aus 80 Gew.-% 2,4- und 20 Gew.-% 2,6-Dinitrotoluol in den Autoklaven geleitet, wobei die Temperatur im Reaktor auf 215°C anstieg. Bei dieser Temperatur und einem Druck von 20 bar wurde bis zur Erschöpfung des Katalysators (50 Stunden) hydriert, wobei kontinuierlich das resultierende Verfahrensprodukt (Gemisch aus Diamin und Wasser) entnommen wurde. Die Ausbeute an Diaminotoluolen entsprach 98,7 % der Theorie, bezogen auf eingesetzte Dinitrotoluole. Außerdem fielen 1,1 Gew.-% teerartige Nebenprodukte und 0,2 Gew.-% sogenannte Niedrigsieder an.

Der so verwendete Katalysator zeigte gegenüber solchen des Standes der Technik bei höherer Arbeitstemperatur eine ebensogute Selektivität. Die höhere Arbeitstemperatur gestattet die Abführung der Reaktionswärme auf höherem Temperaturniveau und ist damit wirtschaftlicher.

Außerdem ließ sich eine Legierung wie sie gemäß a) in dem Induktionsofen erschmolzen wurde, nach Abkühlung in größeren Stücken nicht auf konventionelle Weise in einer Sieb-Kugelmühle vermahlen.

Beispiel 5

a) Herstellung und Verdüsung einer Legierung

In einem Induktionsofen wurden 16,1 kg Zink und 140,2 kg Aluminium geschmolzen. Nach dem Erreichen der Liquidustemperatur wurde die Schmelze weiter bis auf 810°C erhitzt und dann in einen beheizten Auslauftrichter mit einem Bodenauslauf von 12 mm Durchmesser gegossen. Der aus dem Bodenauslauf austretende flüssige Metallstrahl wurde in einer konzentrisch angeordneten Ringspaltdüse mit Wasser zerstäubt. Das Wasser wurde mit einem Druck von 52 bar zugeführt, die zugeführte Wassermenge betrug 1800 l, die Verdüsungszeit 5 Minuten. Das verdüste Pulver wurde in einem unterhalb des Verdüsungssystems befindlichen Wassertank aufgefangen und dabei auf 24°C abgekühlt.

Das dem Tank entnommene Material wies eine Restfeuchte von 18 Gew.-% Wasser auf und wurde in einem trockenen Heißluftstrom von 3200 m$^3$/h innerhalb von 15 Minuten getrocknet.

So wurden 153,5 kg Legierungspulver mit folgender Analyse erhalten: 9,7 Gew.-% Zink, 0,24 Gew.-% Sauerstoff, 0,08 Gew.-% Wasser und 89,7 Gew.-% Aluminium.

Das erhaltene Pulver zeigte eine spratzige bis rundliche Kornform, die erhaltene Kornverteilung war:

```
über 400 µm              1,2 Gew.-%

200 bis 400 µm          12,3 Gew.-%

100 bis 200 µm          34,5 Gew.-%

50 bis 100 µm           30,1 Gew.-%

unter 50 µm             21,9 Gew.-%.
```

Das erhaltene Pulver war lagerstabil und konnte problemlos weiterverarbeitet werden.

b) Anwendung des Katalysators

250 g Toluylendiamin (Gemisch aus 65 Gew.-% 2,4- und 35 Gew.-% 2,6-Toluylendiamin) wurden mit 4,7 g des gemäß a) erhaltenen Legierungspulvers und 8,3 g Aluminiumchlorid (wasserfrei) unter Rühren auf 180° C erhitzt. Bei 130° C begann die Entwicklung von Wasserstoff, die nach 35 Minuten beendet war.

In einem Rührautoklaven wurde der Reaktionsansatz anschließend bei Temperaturen von 250 bis 280° C und bei einem Druck von 150 bis 180 bar mit Ethylen umgesetzt. Die Ethylenaufnahme war nach 25 Minuten beendet.

Nach Aufarbeitung mit wäßriger Natronlauge wurde ein Alkylierungsprodukt erhalten, das nach gaschromatographischer Analyse 98 % an dialkylierten Toluylendiaminen enthielt.

Die höhere Aktivität des eingesetzten Katalysators gegenüber solchen des Standes der Technik ergibt sich aus den niedrigeren Temperaturen, kürzeren Zeiten und geringeren Drucken, die anzuwenden sind.

Beispiel 6

a) Herstellung und Verdüsung einer Legierung

In einem Induktionsofen wurden 99 kg Aluminium und 98 kg Nickel geschmolzen. Nach dem Erreichen der Liquidus-Temperatur wurde die erhaltene Schmelze weiter bis auf 1530° C erhitzt und dann über einen Keramiktrichter mit einem in der Mitte angeordneten Bodenauslauf verdüst. Der aus dem Bodenauslaufstein austretende flüssige Metallstrahl von 12 mm Durchmesser wurde mit Druckluft von 4,7 bar, die durch eine konzentrisch angeordnete Ringspaltdüse zugeführt wurde, verstäubt. Die Verdüsungszeit betrug 8,2 Minuten, der Luftverbrauch 1150 m$^3$ (gemessen bei 4,7 bar). Das verdüste Pulver wurde unterhalb des Verdüsungssystems in einem Wassertank aufgefangen und daraus mit einer Temperatur von 22° C entnommen. Das über einer Nutsche vorentwässerte Pulver enthielt eine Restfeuchtigkeit von 17 Gew.-% Wasser, das in einem Luftstrom von 4000 m$^3$/h aus vorgewärmter, trockener Luft innerhalb von 20 Minuten weitestgehend entfernt wurde.

So wurden 189,5 kg Legierungspulver mit folgender Analyse erhalten:
49,6 Gew.-% Nickel, 49,3 Gew.-% Aluminium, 0,65 Gew.-% Sauerstoff und 0,04 Gew.-% Wasser.

Das erhaltene Pulver zeigte eine überwiegend sphärische Kornform, die erhaltene Kornverteilung war:

| | |
|---|---|
| über 400 µm | 16,5 Gew.-% |
| 200 bis 400 µm | 38,6 Gew.-% |
| 100 bis 200 µm | 33,8 Gew.-% |
| 50 bis 100 µm | 9,6 Gew.-% |
| unter 50 µm | 1,5 Gew.-%. |

b) Herstellung eines Katalysators

In entsprechender Weise wie in Beispiel 1b) beschrieben wurden aus dem gemäß Beispiel 6a) erhaltenen Pulver unter Zuhilfenahme von 500 g Natriumhydroxid und 2000 g Wasser ein Katalysator hergestellt. Es wurden 100 g Katalysator (100 %ig), anfallend als wäßriger Schlamm, erhalten.

c) Anwendung des Katalysators

Bei der Anwendung des gemäß Beispiel 6b) erhaltenen Katalysators entsprechend Beispiel 1c) wurden nahezu die gleichen Hydrierergebnisse erhalten. Auch hierbei zeigte sich die größere Aktivität des Katalysators im Vergleich zu denen des Standes der Technik vor allem in der kürzeren Zeit die zu seiner Herstellung notwendig war.

# EP 0 437 788 B1

**Patentansprüche**

1. Verfahren zur Herstellung von pulverförmigen Aluminiumlegierungen, die eine katalytische Wirkung aufweisen oder als Vorläufer von Katalysatoren dienen, wobei man eine flüssige Aluminiumlegierung mit einer Temperatur von 50 bis 500°C über ihrem Schmelzpunkt mit Wasser und/oder einem Gas verdüst und abkühlt.

2. Verfahren nach Anspruch 1, dadurch gekennzeichnet, daß die Aluminiumlegierung 50 bis 99 Gew.-% Aluminium enthält.

3. Verfahren nach Ansprüchen 1 und 2, dadurch gekennzeichnet, daß die Aluminiumlegierung ein oder mehrere Nebengruppenelemente enthält.

4. Verfahren nach Ansprüchen 1 bis 3, dadurch gekennzeichnet, daß man die Verdüsung bei einer Temperatur von 150 bis 250°C über dem Schmelzpunkt der Legierung durchführt.

5. Verfahren nach Ansprüchen 1 bis 4, dadurch gekennzeichnet, daß man zur Verdüsung pro kg der Aluminiumlegierung 5 bis 15 l Wasser und/oder 0,5 bis 10 m$^3$ Gas (gemessen bei dem in der Verdüsungsapparatur herrschenden Druck) einsetzt.

6. Verfahren nach Ansprüchen 1 bis 5, dadurch gekennzeichnet, daß die Temperatur nach der Abkühlung unter 50°C liegt.

7. Verfahren nach Ansprüchen 1 bis 6, dadurch gekennzeichnet, daß man gegebenenfalls nach der Abkühlung noch vorhandenes Wasser ganz oder bis auf einen Gehalt von unter 1 Gew.-% entfernt.

8. Verwendung von pulverförmigen Aluminiumlegierungen hergestellt gemäß Ansprüchen 1 bis 7 als Katalysatoren für die Alkylierung aromatischer Verbindungen.

9. Verwendung von pulverförmigen Aluminiumlegierungen hergestellt gemäß Ansprüchen 1 bis 7 zur Herstellung von Hydrierkatalysatoren.

**Claims**

1. Process for producing powdered aluminium alloys which have a catalytic action or act as precursors of catalysts, in which a liquid aluminium alloy is atomized with water and/or a gas at a temperature of 50 to 500°C above its melting point and cooled.

2. Process according to Claim 1, characterized in that the aluminium alloy contains 50 to 99% by weight of aluminium.

3. Process according to Claims 1 and 2, characterized in that the aluminium alloy contains one or more subgroup elements.

4. Process according to Claims 1 to 3, characterized in that the atomization is carried out at a temperature of 150 to 250°C above the melting point of the alloy.

5. Process according to Claims 1 to 4, characterized in that 5 to 15 l of water and/or 0.5 to 10 m$^3$ of gas (measured at the pressure prevailing in the atomization apparatus) are used for the atomization per kg of the aluminium alloy.

6. Process according to Claims 1 to 5, characterized in that the temperature after cooling is below 50°C.

7. Process according to Claims 1 to 6, characterized in that any water still present after cooling is removed completely or to a content of below 1% by weight.

8. Use of powdered aluminium alloys produced in accordance with Claims 1 to 7 as catalysts for the alkylation of aromatic compounds.

9

9. Use of powdered aluminium alloys produced in accordance with Claims 1 to 7 for producing hydrogenation catalysts.

**Revendications**

1. Procédé pour la préparation d'alliages d'aluminium en poudre possédant une activité catalytique ou utilisables en tant que matières premières de la préparation de catalyseurs, dans lequel on éjecte et on refroidit à l'aide d'eau et/ou d'un gaz un alliage d'aluminium liquide à une température de 50 à 500°C au-dessus de son point de fusion.

2. Procédé selon la revendication 1, caractérisé en ce que l'alliage d'aluminium contient de 50 à 99 % en poids de ce métal.

3. Procédé selon les revendications 1 et 2, caractérisé en ce que l'alliage d'aluminium contient un ou plusieurs éléments des sous-groupes.

4. Procédé selon les revendications 1 à 3, caractérisé en ce que l'éjection est réalisée à une température de 150 à 250°C au-dessus du point de fusion de l'alliage.

5. Procédé selon les revendications 1 à 4, caractérisé en ce que, pour l'éjection et pour 1 kg de l'alliage d'aluminium, on utilise de 5 à 15 l d'eau et/ou de 0,5 à 10 m$^3$ de gaz (mesure à la pression régnant dans l'appareil d'éjection).

6. Procédé selon les revendications 1 à 5, caractérisé en ce que, après refroidissement, la température est inférieure à 50°C.

7. Procédé selon les revendications 1 à 6, caractérisé en ce que l'eau encore présente après le refroidissement est éventuellement éliminée en totalité ou jusqu'à une teneur résiduelle inférieure à 1 % en poids.

8. Utilisation des alliages d'aluminium en poudre préparés selon les revendications 1 à 7, en tant que catalyseurs de l'alkylation de composés aromatiques.

9. Utilisation des alliages d'aluminium en poudre préparés selon les revendications 1 à 7 pour la préparation de catalyseurs d'hydrogénation.